# EUROPÄISCHE PATENTSCHRIFT

(11) **EP 0 623 668 B1**
(45) Veröffentlichungstag und Bekanntmachung des Hinweises auf die Patenterteilung: **22.03.2000**
(21) Anmeldenummer: 94106879.3
(22) Anmeldetag: 03.05.1994
(51) Int. Cl.: C11D 1/65, C11D 10/04, C11D 9/26, C11D 17/00, C11D 3/37, A61K 7/50

(54) **Stückförmige Körperreinigungsmittel**
Body cleansing bar
Agent de lavage corporel sous forme de pièces

(30) Priorität: 04.05.1993 DE 4314678
(43) Veröffentlichungstag der Anmeldung: 09.11.1994
(73) Patentinhaber: DALLI-WERKE WÄSCHE-UND KÖRPERPFLEGE GmbH & Co.KG., D-52224 Stolberg/Rhld. (DE)
(72) Erfinder: Dijkers, Johannes, NL-6371 CX Landgraaf (NL); Zajac, Aleksander, D-52249 Eschweiler (DE); Müller, Wilfried, D-52531 Übach-Palenberg (DE); Vathje, Rainer, D-52223 Stolberg (DE)
(74) Vertreter: Patentanwälte Sternagel & Fleischer

(56) Entgegenhaltungen:
- EP-A- 0 584 692
- WO-A-92/08444
- FR-A- 2 285 452
- GB-A- 1 221 241
- GB-A- 1 296 351
- COSMETICS & TOILETRIES, Bd.106, Nr.7, Juli 1991 Seiten 59 - 65 POLOVSKY STUART B.

## Beschreibung

Die Erfindung betrifft stückförmige Körperreinigungsmittel auf Basis anionischer Tenside, die 0,1 bis 10 Gew.-% eines Gemisches aus
a) C₃₆-Dimer- und/oder C₅₄-Trimerfettsäure und
b) wenigstens einer bei 15 bis 25° C flüssigen kationischen Verbindung, ausgewählt aus der Gruppe der ethoxylierten Methylglucosidhydroxypropyl-trialkylammonium-halogenide, in denen die Summe der C-Atome der Trialkylammoniumgruppe zwischen 12 und 37 liegt, und/oder der quartäre Ammoniumgruppen enthaltenden Polydimethylsiloxane mit einem mittleren Molekulargewicht zwischen 1500 und 3000
enthalten. Stückförmige Körperreinigungsmittel sollen neben guten Reinigungs- und Schaumeigenschaften hautschonend sein und ein Austrocknen der Haut, insbesondere bei häufiger Anwendung der Reinigungsmittel, vermeiden. Zusammensetzungen auf Basis von Fettsäuresalzen irritieren jedoch die Haut insbesondere bei Temperaturen unterhalb von 5° C, Zusammensetzungen auf Basis von synthetischen Tensiden trocknen die Haut aus und bewirken eine rauhe Hautoberfläche. Dementsprechend hat es nicht an Bemühungen gefehlt, die Hauteigenschaften von stückförmigen Körperreinigungsmitteln zu verbessern. So ist aus der europäischen Patentanmeldung EP 227 321 bekannt, daß hydratisierte kationische Polymere, die Seifen in Mengen bis zu 5 % zugesetzt werden, eine Verbesserung der Hautkonditionierung bewirken. Zur Herstellung eines hydratisierten kationischen Polymers wird ein kationisches Polymer entweder mit Wasser vermischt und die erhaltene Mischung zu den übrigen Bestandteilen der Seifenzusammensetzung gegeben oder direkt der wasserhaltigen Seifenmasse zugesetzt. Die Hydratisierung der kationischen Polymere, die 12 bis 24 Stunden dauert, erfordert jedoch große Wassermengen, um das Polymer zu lösen und die resultierende Mischung fließfähig zu halten. Darüber hinaus macht die gleichmäßige Verteilung des hydratisierten kationischen Polymeren in der Seifenzusammensetzung den Einsatz von Rühraggregaten erforderlich.

Zur Verbesserung der Gebrauchseigenschaften von stückförmigen Seifen ist ferner die Mitverwendung von Dicarbonsäuren bekannt. Solche Seifen zeigen jedoch beim Extrudieren eine brüchige Konsistenz und sind nicht ausreichend plastisch verformbar. Dies wird auf den hohen Schmelzpunkt der Dicarbonsäuren und die schlechte Vermischbarkeit mit der Seifenmasse während der Herstellung des Seifengrundkörpers zurückgeführt. Um die durch die Inhomogenität hervorgerufene Rißbildung zu verhindern, wird in der deutschen Patentanmeldung DE 32 46 796 die Einarbeitung einer Dicarbonsäure mit 2 bis 20 C-Atomen in Kombination mit einem ethoxylierten Tensid in Seifenmassen vorgeschlagen.

Darüber hinaus sind aus der österreichischen Patentschrift AT 322 707 Toilettenwaschmittelstücke mit einem Gehalt einer quaternären Polyethylenoxidammoniumverbindung bekannt.

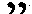 In dem Dokument FR - A - 2285452 werden Zusätze zu Seifen beansprucht, die der Versumpfung von Seifenstücken entgegenwirken, dem Seifenkörper eine verbesserte Härte verleihen und die Oberflächenstruktur verbessern. Diese Zusätze sind Natriumchlorid / Natriumsulfat und dimere Linolsäure sowie Mischungen von diesen Substanzen.

In der GB - A - 1221241 werden oberflächenaktive Kompositionen mit Additiven, die die Hautverträglichkeit verbessern sollen, beansprucht. Zusätzlich wird eine tierexperimentelle Methode zur Bestimmung der Hautverträglichkeit beschrieben. Die beanspruchten Additive sind Derivate von Dicarbonsäuren, z.B. Amide und Ester.

In der Zeitschrift Cosmetics & Toiletries Vol. 106, July 1991, S. 59 bis 65 von Stuart B. Polovsky werden in dem Artikel 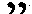 An Alkoxylated Methyl Glucoside Quaternary For Conditioning Hair and Skin" die Herstellung, die Eigenschaften und die Verwendung des ethoxylierten Methylglucosidhydroxypropyl-dimethyldodecylammoniumchlorides (= Gluquat 100 der Firma Amerchol) beschrieben. Dort werden besonders die niedrige Toxizität, die Verträglichkeit mit anionischen Verbindungen und die hohe Akzeptanz von Methylglucose-verbindungen hervorgehoben.

Die der Erfindung zugrundeliegende Aufgabe bestand in der Bereitstellung stückförmiger Körperreinigungsmittel auf Basis von anionischen Tensiden, die sich innerhalb kurzer Zeit homogen in anionische Tenside enthaltende Zusammensetzungen einarbeiten lassen und gegenüber bekannten stückförmigen Körperreinigungsmitteln verbesserte Gebrauchseigenschaften bei mindestens gleichwertigen technischen Eigenschaften, beispielsweise Rißbildung und/oder Festigkeit, besitzen.

Es wurde gefunden, daß diese an stückförmige Körperreinigungsmittel gestellten Anforderungen von anionische Tenside enthaltenden Zusammensetzungen erfüllt werden, denen eine Mischung aus
a) C₃₆-Dimer- und/oder C₅₄-Trimerfettsäure und
b) wenigstens einer bei 15 bis 25° C flüssigen kationischen Verbindung, ausgewählt aus der Gruppe der ethoxylierten Methylglucosidhydroxypropyl-trialkylammonium-halogenide, in denen die Summe der C-Atome der Tri-alkylammoniumgruppe zwischen 12 und 37 liegt, und/oder der quartäre Ammoniumgruppen enthaltenden Polydimethylsiloxane mit einem mittleren Molekulargewicht zwischen 1500 und 3000
   in einer bestimmten Menge zugesetzt wurde.

Erfindungsgegenstand sind dementsprechend stückförmige Körperreinigungsmittel enthaltend 65 bis 95 Gew.-% anionische Tenside sowie bis zu 25 Gew.-% Wasser, welche dadurch gekennzeichnet sind, daß sie 0,1 bis 10 Gew.-% eines Gemisches aus
a) C₃₆-Dimer- und/oder C₅₄-Trimerfettsäure und
b) wenigstens einer bei 15 bis 25° C flüssigen kationischen Verbindung, ausgewählt aus der Gruppe der ethoxylierten Methylglucosidhydroxypropyl-trialkylammonium-halogenide, in denen die Summe der C-Atome der Trialkylammoniumgruppe zwischen 12 und 37 liegt, und/oder der quartäre Ammoniumgruppen enthaltenden Polydimethylsiloxane mit einem mittleren Molekulargewicht zwischen 1500 und 3000
   enthalten.

Bevorzugte erfindungsgemäße Körperreinigungsmittel enthalten 0,4 bis 2 Gew.-% eines Gemisches aus den Komponenten a) und b). Das Gewichtsverhältnis der Komponente a) zu Komponente b) liegt vorzugsweise zwischen 1 : 10 und 10 : 1, besonders bevorzugt zwischen 1 : 2 und 2 : 1.

Die durch thermische En-Addition aus ungesättigten C₁₈-Fettsäuren, beispielsweise aus Ölsäure und/oder Linolsäure zugängliche C₃₆-Dimerfettsäure bzw. C₅₄-Trimerfettsäure kann als reine Dimerfettsäure bzw. reine Trimerfettsäure sowie als Mischung aus Dimer- und Trimerfettsäure vorliegen. Vorzugsweise werden C₃₆-Dimerfettsäure und/oder C₅₄-Trimerfettsäure mit einer Jodzahl zwischen 5 und 110 eingesetzt, beispielsweise die im Handel von Unichema International unter der Bezeichnung Pripol® 1009 erhältliche C₃₆-Dimerfettsäure oder die unter der Bezeichnung Pripol® 1040 erhältliche Mischung aus 78 Gew.-% C₅₄-Trimerfettsäure und 22 Gew.-% C₃₆-Dimerfettsäure.

Als ethoxylierte Methylglucosidhydroxypropyl-trialkylammoniumhalogenide, in denen die Summe der C-Atome der Trialkylammoniumgruppe zwischen 12 und 37 liegt, werden vorzugsweise ethoxylierte Methylglucosidhydroxypropyl-dimethyl-dodecyl-ammoniumchloride, beispielsweise Gluquat® 100 von Amerchol, eingesetzt. Als kationische, bei Raumtemperatur, das heißt zwischen 15 und 25° C flüssige Verbindung aus der Gruppe der quartäre Ammoniumgruppen enthaltenden Polydimethylsiloxane mit einem mittleren Molekulargewicht zwischen 1.500 und 3.000 eignet sich beispielsweise Abil-Quat 3272 der Firma Goldschmidt AG.

Mischungen aus Komponente a) und Komponente b) sind bei Raumtemperatur, das heißt zwischen 15 und 25° C flüssig, klar, einphasig, in Wasser löslich bzw. dispergierbar und wirken auf lipophile Substanzen emulgierend. Diese Mischungen lassen sich innerhalb kurzer Zeit homogen in Massen auf Basis von anionischen Tensiden einarbeiten.

Die erfindungsgemäßen stückförmigen Körperreinigungsmittel zeigen gute Schaum- und Abspüleigenschaften, neigen nicht zur Rißbildung und haben gute Eigenschaften hinsichtlich Festigkeit, Schaumstruktur und Verschleimung. Darüber hinaus zeichnen sie sich durch ein sehr gutes Hautgefühl aus.

Die Einarbeitung eines Gemisches aus
a) C₃₆-Dimer- und/oder C₅₄-Trimerfettsäure und
b) wenigstens einer bei 15 bis 25° C flüssigen kationischen Verbindung, ausgewählt aus der Gruppe der ethoxylierten Methylglucosidhydroxypropyl-trialkylammonium-halogenide, in denen die Summe der C-Atome der Trialkylammoniumgruppe zwischen 12 und 37 liegt, und/oder der quartäre Ammoniumgruppen enthaltenden Polydimethylsiloxane mit einem mittleren Molekulargewicht zwischen 1500 und 3000
   in Zusammensetzungen auf Basis anionischer Tenside erfolgt in an sich bekannter Weise durch Mischen, indem das Gemisch zu den übrigen Bestandteilen des erfindungsgemäßen Mittels oder die übrigen Bestandteile in beliebiger Reihenfolge nacheinander oder zusammen zu dem Gemisch gegeben werden. Vorzugsweise wird jedoch das Gemisch zu den übrigen Bestandteilen des erfindungsgemäßen Mittels gegeben. Während des Mischvorgangs liegt die Temperatur üblicherweise zwischen 15 und 95° C.

Als anionische Tenside, die natürlichen und/oder synthetischen Ursprungs sind, eignen sich Alkali- und/oder Erdalkalisalze von geradkettigen und/oder verzweigtkettigen C₈₋₂₀-Monocarbonsäuren, beispielsweise Caprylsäure, Caprinsäure, Laurinsäure, Myristinsäure, Palmitinsäure, Stearinsäure, Palmtoleinsäure, Ölsäure, Ricinolsäure, Linolsäure, Linolensäure und durch Oxydation von Paraffin oder durch Oxosynthese hergestellte Monocarbonsäuren. Vorzugsweise werden Alkalisalze von C₈₋₂₀-Fettsäuren, besonders bevorzugt Alkalisalze von C₁₂₋₁₈-Fettsäuren, beispielsweise Laurinsäure, Myristinsäure, Palmitinsäure, Stearinsäure, Ölsäure sowie Gemische dieser Fettsäuren mit gegebenenfalls geringen Anteilen weiterer Fettsäuren eingesetzt.

Als anionische Tenside synthetischen Ursprungs eignen sich ferner Alkalisalze von C₁₄₋₁₈-Olefinsulfonaten, Alkalisalze von Sulfobernsteinsäuremono- und/oder Sulfobernsteinsäuredialkylestern, beispielsweise Sulfobernsteinsäuremonolaurylester in Form des Dinatriumsalzes, Alkalisalze von C₁₂₋₁₈-Fettalkoholsulfaten, beispielsweise Kokosalkylsulfat, Natriumsalz, Natrium-C₁₂₋₁₈-fettalkylisethionate, beispielsweise Natriumlauryl-, Natriummyristyl- und/oder Natriumkokosalkylisethionat, und/oder Natriumtaurid.

Anionische Tenside natürlichen und/oder synthetischen Ursprungs können zusammen mit nichtionischen Tensiden, beispielsweise ethoxylierten C₁₂₋₁₈-Fettalkoholen und/oder C₁₀₋₁₈-Fettalkylpolyglycosiden, eingesetzt werden, wobei insbesondere 5 bis 30 Gew.-% des Anteils an anionischen Tensiden durch nichtionische Tenside ersetzt wird.

Die erfindungsgemäßen stückförmigen Körperreinigungsmittel enthalten darüber hinaus bis zu 25 Gew.-%, im allgemeinen 5 bis 25 Gew.-%, vorzugsweise 10 bis 18 Gew.-% Wasser und gegebenenfalls übliche Zusätze wie Plastifiziermittel, beispielsweise C₁₆₋₁₈-Fettalkohole und Paraffin, Gerüststoffe wie Polysaccharide, beispielsweise Mais und Stärke, Duftstoffe, Füller, pH-Wert-Regulatoren, Pigmente und Farbstoffe. Der Gehalt dieser fakultativen Hilfsstoffe liegt zwischen 1 und 25 Gew.-%, vorzugsweise zwischen 4 und 25 Gew.-%.

### Beispiele

### 1. Herstellung stückförmiger Körperreinigungsmittel auf Basis von Fettsäuresalzen

Talgfettsäure und Kokosfettsäure im Gewichtsverhältnis 4 : 1 wurden bei 95°C mit Natronlauge verseift, und Natriumchlorid in der Menge zugefügt, daß ein Gehalt von 0,4 Gew. % Natriumchlorid im geformten Seifenstück resultierte. Die erhaltene Seifenmasse wurde im Vakuum auf einen Gesamtfettsäuregehalt von 80 Gew. % getrocknet und anschließend mit den übrigen Additiven, in der in Tabelle 1 angegebenen Reihenfolge, bei 25°C vermischt. Den Seifenmassen nach den Beispielen D (erfindungsgemäß) und E wurde zuletzt eine flüssige Mischung aus C₃₆-Dimerfettsäure und einer kationischen Verbindung zugesetzt. In einem Pilierwalzwerk wurde über eine Vakuumstrangpresse ein Seifenstrang erzeugt, aus welchem mittels einer Seifenpresse ein geformtes Seifenstück herstellt wurde.

**Tabelle 1**

| Bestandteile | Beispiel | | | | |
|---|---|---|---|---|---|
| | A | B | C | D | E |
| | (Angaben in Gewichtsprozent) | | | | |
| Talgfettsäure, Natriumsalz | 65,92 | 65,92 | 65,92 | 65,92 | 65,92 |
| Kokosfettsäure, Natriumsalz | 16,48 | 16,48 | 16,48 | 16,48 | 16,48 |
| Wasser | 13,5 | 13,00 | 13,00 | 13,00 | 13,00 |
| Glycerin | 1,40 | 1,40 | 1,40 | 1,40 | 1,40 |
| Riechstoff | 1,35 | 1,35 | 1,35 | 1,35 | 1,35 |
| Jojobaöl | 0,50 | 0,50 | 0,50 | 0,50 | 0,50 |
| Titandioxid | 0,40 | 0,40 | 0,40 | 0,40 | 0,40 |
| C₃₆-Dimerfettsäure¹⁾ | - | 0,50 | - | 0,30 | 0,30 |
| ethoxyliertes Methylglucosidhydroxypropyl-dimethyldodecylammoniumchlorid²⁾ | - | - | 0,50 | 0,20 | - |
| Hydroxyethylcellulose-Kokosalkyldimethylammoniumchlorid³⁾ | - | - | - | - | 0,30 |
| Ethylendiamintetraessigsäure | 0,05 | 0,05 | 0,05 | 0,05 | 0,05 |

| | | | | | |
|---|---|---|---|---|---|
| ¹⁾ Pripol® 1009 der Firma Unichema | | | | | |
| ²⁾ Gluquat® 100 der Firma Amerchol | | | | | |
| ³⁾ Crodacel® QM der Firma Croda | | | | | |

Die erhaltenen stückförmigen Seifenmassen wurden hinsichtlich Rißbildung, Verschleimung, Festigkeit, Schaumstruktur und Hautgefühl geprüft.

### Rißbildung

Die Seifenstücke wurden mit Drähten aufgespießt und zur Hälfte in ein Wasserbad von 20° C gehängt. Nach 4 Stunden wurde das Wasser abgelassen und die Seifenstücke an der Luft getrocknet. Danach wurde jedes Seifenstück nach seinem Rißbild mit den Noten 1 bis 6 beurteilt. Dabei wurde folgendes Beurteilungsschema verwendet:
- 1 =: keine Risse oder Haarrisse an den Seifennähten
- 2 =: leichter Riß in den Seifennähten
- 3 =: breiterer Riß in den Seifennähten
- 4 =: leichter Riß über die gesamte Nahtfläche
- 5 =: spaltförmiger Riß über die gesamte Nahtfläche
- 6 =: tiefer oder breiter Spalt über die gesamte Nahtfläche

Mit den Seifenstücken wurden folgende Ergebnisse erhalten:

| Beispiel | A | B | C | D | E |
|---|---|---|---|---|---|
| Rißbildung | 1 | 4 | 3 | 1 | 2 |

### Verschleimung

Die Verschleimung ist ein Maß für die Wassereindringung in Seifenstücke. Wie bei der Ermittlung der Rißbildung wurden die Seifenstücke 4 Stunden zur Hälfte in ein Wasserbad mit einer Temperatur von 20° C gehängt. Mit einer Schieblehre wurde dann die Dicke der gequollenen Seife gemessen. Die Schieblehre wurde dann bis zum Widerstand zusammengeschoben. Sobald ein Widerstand zu spüren war, wurde dieser Wert abgelesen. Er repräsentierte die Dicke des festen, das heißt nicht hydratisierten Seifenkörpers. Aus der Differenz ergibt sich die Dicke der verschleimten Seifenmasse, die in Prozent von der nicht gequollenen Seifendichte berechnet wurde. Bei allen Seifenstücken lagen die Werte zwischen 18 bis 21 %.

### Festigkeit

Die Festigkeit stellt das Verhältnis zwischen der Dicke des Seifenkörpers vor der Wasseraufnahme und der Dicke des harten Seifenkerns nach der Hydratation dar. Die Dicke der harten Seifenkerne lag zwischen 89 % und 93 % der ursprünglichen Dicke der nicht hydratisierten Seife.

### Schaumstruktur

Die Schaumstruktur wurde von 5 in der Beurteilung von Schäumen geübten Personen ermittelt. Hier resultierte folgendes Ergebnis:

| Beispiel | A | B | C | D | E |
|---|---|---|---|---|---|
| Schaumstruktur | 3 | 3 | 3 | 1 | 2 |

wobei
- 1 =: feinporiger, sahniger Schaum
- 2 =: feinporiger Schaum
- 3 =: grobblasiger Schaum
bedeutet.

### Hautgefühl

Das Hautgefühl wurde von 15 Testpersonen, die häufig diese Art von Testen durchführen, beurteilt. Bei dem erfindungsgemäßen Beispiel D wurde das Hautgefühl signifikant besser beurteilt als bei den Beispielen A, B, C und E. Nach dem Waschen mit der Seife gemäß Beispiel D empfanden die Testpersonen eine spürbar bessere Hautglätte und ein deutlich besseres Feuchtigkeitsgefühl.

### 2. Herstellung stückförmiger Körperreinigungsmittel auf Basis anionischer Tenside synthetischen Ursprungs

Die Herstellung der in Tabelle 2 angegebenen Zusammensetzungen F und G erfolgte analog Beispiel 1, in dem zu den anionischen Tensiden die übrigen Bestandteile in der in Tabelle 2 angegebenen Reihenfolge gemischt wurden. Der Mischung nach Beispiel G wurde zuletzt eine flüssige Mischung aus C₃₆-Dimerfettsäure und einer kationischen Verbindung zugesetzt.

**Tabelle 2**

| Bestandteile | Beispiel | |
|---|---|---|
| | F | G |
| | (Angaben in Gewichtsprozent) | |
| Gemisch aus Sulfobernsteinsäuremonolaurylester, Dinatriumsalz, Kokosalkylsulfat, Natriumsalz und C_{16/18}-Fettalkohol¹⁾ | 90 | 90 |
| Wasser | 4,95 | 4,45 |
| Phosphorsäure | 3,40 | 3,40 |
| Riechstoff | 1,35 | 1,35 |
| Titandioxid | 0,3 | 0,3 |
| C₃₆-Dimerfettsäure²⁾ | - | 0,3 |
| ethoxyliertes Methylglucosidhydroxypropyldimethyl-dodecylammoniumchlorid³⁾ | - | 0,20 |

| | | |
|---|---|---|
| ¹⁾ Zetesap® 813 A der Firma Zschimmer & Schwarz | | |
| ²⁾ Pripol® 1009 der Firma Unichema | | |
| ³⁾ Gluquat® 100 der Firma Amerchol | | |

Die Schaumeigenschaften (Cremigkeit und Feinblasigkeit), die Hautglätte sowie das Feuchtigkeitsgefühl wurden bei der erfindungsgemäßen Zusammensetzung G deutlich besser beurteilt als bei der Zusammensetzung F.

## Patentansprüche

1. Stückförmiges Körperreinigungsmittel enthaltend 65 bis 95 Gew.-% anionische Tenside sowie bis zu 25 Gew.-% Wasser dadurch gekennzeichnet, daß dieses 0,1 bis 10 Gew.-% eines Gemisches aus
a) C₃₆-Dimer- und/oder C₅₄-Trimerfettsäure und
b) wenigstens einer bei 15 bis 25° C flüssigen kationischen Verbindung, ausgewählt aus der Gruppe der ethoxylierten Methylglucosidhydroxypropyl-trialkylammonium-halogenide, in denen die Summe der C-Atome der Trialkylammoniumgruppe zwischen 12 und 37 liegt, und/oder der quartäre Ammoniumgruppen enthaltenden Polydimethylsiloxane mit einem mittleren Molekulargewicht zwischen 1500 und 3000
enthält.

2. Stückförmiges Körperreinigungsmittel nach Anspruch 1, dadurch gekennzeichnet, daß dieses 0,4 bis 2 Gew.-% eines Gemisches aus Komponente a) und Komponente b) enthält.

3. Stückförmiges Körperreinigungsmittel nach einem oder beiden der Ansprüche 1 bis 2, dadurch gekennzeichnet, daß das Gewichtsverhältnis der Komponente a) zu Komponente b) zwischen 1 : 10 und 10 : 1, vorzugsweise zwischen 1 : 2 und 2 : 1 liegt.

4. Stückförmiges Körperreinigungsmittel nach Anspruch 1, dadurch gekennzeichnet, daß die Komponente b) ein ethoxyliertes Methylglucosidhydroxypropyl-dimethyldodecylammoniumchlorid ist.

## Claims

1. Body cleansing bar composition comprising 65 to 95 percent by weight of anionic detergents and up to 25 percent by weight water, characterized by comprising 0.1 to 10 percent by weight of a blend of
a) C₃₆-dimeric and/or C₅₄-trimeric fatty acid, and
b) at least one cationic compound which is liquid at 15 to 25°C, selected from the group of ethoxylated methylglucosidehydroxypropyltrialkylammonium halides, wherein the sum of C-atoms of the trialkylammonium radical is between 12 and 37, and/or quaternary ammonium radicals containing polydimethylsiloxanes having an average molecular weight between 1,500 and 3,000.

2. Body cleansing bar composition according to claim 1, characterized by comprising 0.4 to 2 percent by weight of a blend of component a) and component b).

3. Body cleansing bar composition according to one or both of claims 1 to 2 characterized in that the weight ratio of component a):component b) is between 1:10 and 10:1, preferably between 1:2 and 2:1.

4. Body cleansing bar composition according to claim 1, characterized in that component b) is an ethoxylated methylglucosidehydroxy-propyldimethyldodecylammoniumchloride.

## Revendications

1. Agent de nettoyage corporel en morceaux contenant 65 à 95% en poids de tensio-actifs anioniques, ainsi que jusqu'à 25% d'eau, caractérisé en ce que celui-ci contient 0,1 à 10% en poids d'un mélange constitué de
a) un acide gras dimère en C₃₆ et/ou trimère en C₅₄ et
b) au moins un composé cationique liquide entre 15 et 25°C, choisi dans le groupe des halogénures éthoxylés de méthylglucoside-hydroxypropyl-trialkylammonium, dans lesquels la somme des atomes de C du groupe trialkylammonium est comprise entre 12 et 37, et/ou du polydiméthylsiloxane contenant des groupes ammonium quaternaire ayant une masse moléculaire
moyenne comprise entre 1500 et 3000.

2. Agent de nettoyage corporel en morceaux selon la revendication 1, caractérisé en ce que celui-ci contient 0,4 à 2% en poids d'un mélange de composant a) et de composant b).

3. Agent de nettoyage corporel en morceaux selon l'une ou les deux revendications 1 à 2, caractérisé en ce que le rapport pondéral du composant a) au composant b) est compris entre 1:10 et 10:1, de préférence entre 1:2 et 2:1.

4. Agent de nettoyage corporel en morceaux selon la revendication 1, caractérisé en ce que le composant b) est un chlorure de méthylglucoside-hydroxypropyl-diméthyl-dodécyl-ammonium.
